# EUROPÄISCHE PATENTSCHRIFT

(11) **EP 3 328 324 B1**
(45) Veröffentlichungstag und Bekanntmachung des Hinweises auf die Patenterteilung: **15.05.2019**
(21) Anmeldenummer: 16753270.4
(22) Anmeldetag: 27.07.2016
(51) Int. Cl.: A61F 2/64, A61F 2/66, A61F 5/01, F16F 3/06

(54) **GELENK FÜR EINE ORTHOPÄDIETECHNISCHE EINRICHTUNG**
JOINT FOR AN ORTHOPAEDIC DEVICE
ARTICULATION POUR UN DISPOSITIF ORTHOPÉDIQUE

(30) Priorität: 28.07.2015 DE 102015112283
(43) Veröffentlichungstag der Anmeldung: 06.06.2018
(73) Patentinhaber: Ottobock SE & Co. KGaA, 37115 Duderstadt (DE)
(72) Erfinder: HOCHMANN, David, 48565 Steinfurt (DE); LÜRSSEN, Marcus, 37073 Göttingen (DE); SCHILLING, Matthias, 37345 Weissenborn-Lüderode (DE)
(74) Vertreter: Gramm, Lins & Partner Patent- und Rechtsanwälte PartGmbB
(86) Internationale Anmeldenummer: PCT/EP2016/067937
(87) Internationale Veröffentlichungsnummer: WO 2017/017151

(56) Entgegenhaltungen:
- DE-A1- 2 916 446
- DE-A1-102010 014 334
- DE-A1-102013 101 467
- DE-C- 322 048
- DE-C- 841 194
- US-A1- 2007 075 471

## Beschreibung

Die Erfindung betrifft ein Gelenk für eine orthopädietechnische Einrichtung, insbesondere eine Orthese oder eine Prothese, wobei das Gelenk ein erstes Element, wenigstens ein Federelement und ein zweites Element aufweist, das an dem ersten Element gegen eine von dem wenigstens einen Federelement in wenigstens einer ersten Richtung aufgebrachte Kraft schwenkbar gelagert ist. Ein derartiges Gelenk ist beispielsweise in Form eines Knöchelgelenkes für eine Beinorthese aus der DE 10 2010 014 334 A1 bekannt. Derartige Knöchelgelenke können bei Bein- oder Unterschenkelorthesen verwendet werden. Dabei kann es aus therapeutischen Gründen sinnvoll sein, die Länge der Schwenkbewegung, also den maximal möglichen Schwenkwinkel, des zweiten Elementes relativ zum ersten Element zu begrenzen und beispielsweise in einer oder beiden Schwenkrichtungen jeweils einen Anschlag vorzusehen. Um ein zu hartes Anschlagen an diesen Anschläge zu vermeiden, sind diese Anschläge in der Regel federbelastet und damit gedämpft ausgeführt. Diese Federung sorgt zudem dafür, dass ein Verschwenken des Gelenkes für die orthopädietechnische Einrichtung nur möglich ist, wenn die durch die Feder aufgebrachte Kraft überwunden wird. Auch dies kann zu Rehabilitations- und Trainingszwecken sinnvoll sein.

Insbesondere bei der Verwendung des Gelenkes als Knöchelgelenk aber auch bei anderen Anwendungsfeldern muss dabei das Federelement eine ausreichend große Federkraft und Federkonstante aufweisen und gleichzeitig möglichst wenig Bauraum benötigen. Bei der aus dem Stand der Technik bekannten Ausführungsform wird dies über eine Tellerfederanordnung, die insbesondere als Stapel von übereinander angeordneten Tellerfederelementen ausgebildet ist, erreicht. Diese weisen eine hohe Federkraft und verglichen mit herkömmlichen Blatt- oder Schraubenfedern gleicher Federstärke einen relativ geringen Bauraum auf. Nachteilig ist jedoch, dass Tellerfederanordnungen einerseits kostenintensiv und andererseits aufwändig zu fertigen und zu montieren sind. Zudem besteht die Gefahr, dass eine oder mehrere der Tellerfedern unter beispielsweise zu hoher Belastung oder aufgrund von Ermüdungserscheinungen brechen. Dies hätte eine schlagartige Reduzierung der Federkraft und damit der Dämpfung des Gelenks zur Folge, wodurch sich der Träger der Orthese, in der das Gelenk verbaut wird, erschrecken und schlimmstenfalls stolpern könnte.

Der Erfindung liegt daher die Aufgabe zu Grunde, ein gattungsgemäßes Gelenk so weiter zu entwickeln, dass die beschriebenen Nachteile vermindert oder gänzlich verhindert werden.

Die Erfindung löst die gestellte Aufgabe durch ein gattungsgemäßes Gelenk für eine orthopädietechnische Einrichtung, insbesondere eine Orthese oder eine Prothese, wobei das Gelenk ein erstes Element, wenigstens ein Federelement und ein zweites Element aufweist, das an dem ersten Element gegen eine von dem wenigstens einen Federelement in wenigstens einer ersten Richtung aufgebrachte Kraft schwenkbar gelagert ist, wobei sich das Gelenk dadurch auszeichnet, dass das wenigstens eine Federelement wenigstens zwei Schraubenfedern aufweist, die jeweils aus einem Federband mit einer längeren Querschnittsseite hochkant zur Federachse gewickelt sind und derart ineinander geschraubt sind, dass die längeren Querschnittsseiten einen in unterschiedliche Richtungen von 90° abweichenden Winkel gegen die Federachse aufweisen und dass die Federbänder aneinander anliegen.

Derartige Federelemente werden beispielsweise unter der Bezeichnung "Schraubentellerfeder" von der Dr. Werner Röhrs GmbH & Co. KG vertrieben. Sie kommen beispielsweise in Wasserstoff-Brennstoffzellen mit hoher Energiedichte, wie sie beispielsweise in der Satelliten-Raumfahrt oder bei Unterseeboten verwendet werden, zum Einsatz. Zudem können sie in Werkzeugmaschinen und Werkzeugspannern oder in Streckblasmaschinen bei der PET-Formung verwendet werden. Der Erfindung liegt nun die überraschende Erkenntnis zu Grunde, dass auch die völlig unterschiedlichen Anforderungen der vorliegenden Anwendung als Gelenk für eine orthopädietechnische Einrichtung gegenüber den bekannten Anwendungen durch derartige Federelemente gelöst werden.

Erfindungsgemäß verfügt das Federband über einen Querschnitt, der eine längere Querschnittseite und entsprechend eine kürzere Querschnittseite aufweist. Der Querschnitt ist vorteilhafterweise rechteckig. Die vier Seiten bilden die beiden längeren Querschnittsseiten und die beiden kürzeren Querschnittseiten, so dass die längere Querschnittseite und die kürzere Querschnittseite gerade Strecken sind. Alternativ dazu kann es auch möglich sein, den Querschnitt gebogen oder gewölbt oder unregelmäßig auszuführen, so dass die längeren Querschnittsseiten und/oder die kürzeren Querschnittseiten selbst gebogen ausgebildet sind.

Die Federachse erstreckt sich in der Längsrichtung der wenigstens zwei Schraubenfedern. Erfindungsgemäß sollen die Schraubenfedern mit der längeren Querschnittsseite hochkant zur Federachse gewickelt sein. Dies bedeutet insbesondere, dass ein Winkel, den die längere Querschnittsseite mit einer Richtung einschließt, die senkrecht auf der Federachse steht und daher als "Lotrechte" bezeichnet wird, vorzugsweise zwischen 45° und -45°, bevorzugt zwischen 30° und -30°, besonders bevorzugt zwischen 20° und -20° liegt. Für den Fall, dass die längere Querschnittsseite nicht gerade, sondern beispielsweise gebogen oder gewölbt ausgebildet ist, gilt dieser Winkel insbesondere am radial inneren Ende der längeren Querschnittsseite relativ zur Federachse.

Dass die Schraubenfeder aus einem Federband gewickelt ist, bedeutet nicht, dass sie auch auf diese Weise hergestellt wird. Damit soll lediglich die Form der Schraubenfeder beschrieben werden. Vorteilhafterweise werden Schraubenfedern auch für Federelemente für Gelenke gemäß der vorliegenden Erfindung zwar auf diese Weise hergestellt, es ist jedoch auch möglich, entsprechende Schraubenfedern die nahezu gleichen Eigenschaften aufweisen, generisch, beispielsweise durch Lasersintern aus Titan herzustellen. Auf diese Weise können auch Konturen hergestellt werden, die durch das tatsächliche Wickein eines Federbandes nicht hergestellt werden können.

Erfindungsgemäß sind die wenigstens zwei Schraubenfedern mit ihrer Längsseite hochkant derart gewickelt, dass die längeren Querschnittsseiten wenigstens einer der Schraubenfedern, bevorzugt beider Schraubenfedern einen von 90° abweichenden Winkel zur Federachse aufweisen. Die längeren Querschnittsseiten der wenigstens zwei Schraubenfedern weichen dabei in unterschiedliche Richtungen von dem rechen Winkel zur Federachse ab. Auf diese Weise ist sichergestellt, dass die wenigstens zwei Schraubenfedern nicht vollflächig aneinander anliegen, wodurch der Federeffekt stark beeinträchtigt oder vollständig aufgehoben würde. Besonders vorteilhafterweise liegen die beiden Schraubenfedern lediglich entlang einer kontakten Linie aneinander an. Wird die Feder belastet, indem sie beispielsweise zusammengedrückt, also gestaucht wird, werden die wenigstens zwei Schraubenfedern ebenfalls gestaucht und der Winkel der längeren Querschnittsseite des Querschnitts des jeweiligen Federbandes der beiden Schraubenfedern relativ zur Federachse verändert sich. Dabei nimmt die Abweichung von dem rechten Winkel zur Federachse vorteilhafterweise mit wachsender Belastung der hier verwendeten Federelemente ab.

Prinzipiell ist es ausreichend, wenn die längere Querschnittsseite nur einer der verwendeten Schraubenfedern von einem Winkel zur Federachse aufweist, der von 90° abweicht. Die jeweils zweite verwendete Schraubenfeder kann so ausgebildet sein, dass die längere Querschnittsseite exakt im rechten Winkel zur Federachse angeordnet ist. Vorteilhafterweise sind jedoch alle verwendeten Schraubenfedern so ausgebildet, dass die längeren Querschnittsseiten einen von 90° abweichenden Winkel gegen die Federachse aufweisen. In diesem Fall ist es vorteilhaft, wenn die Winkel der längeren Querschnittsseiten für unterschiedliche Schraubenfedern in unterschiedliche Richtungen von dem rechten Winkel gegen die Federachse abweichen und/oder unterschiedlich stark von diesem rechten Winkel abweichen. Die tatsächlich gewählten Winkel hängen dabei von der benötigten Federkraft, der Federkennlinie und sonstiger geforderter Anforderungen ab. Es ist auch möglich, den Winkel der längeren Querschnittsseite zur Federachse über die Länge des jeweiligen Federelementes zu variieren und so in unterschiedlichen Bereichen des jeweiligen Federelementes unterschiedlich starke Federkonstanten zu erreichen.

Durch die besondere Art des Federelementes mit zwei ineinander geschraubten Schraubenfedern, die vorteilhafterweise identisch zueinander ausgebildet sind, wird einerseits die Anfälligkeit des Federelementes gegen ein Brechen beispielsweise aufgrund hoher mechanischer Belastungen, verringert. Da die beiden ineinander geschraubten Schraubenfedern jeweils einteilig ausgebildet sind und durch die jeweils andere Schraubenfeder in ihrer Position gehalten werden, hat ein Brechen einer der Schraubenfedern keine Veränderung der jeweils aufgebrachten Federkraft oder das Herauslösen einzelner beschädigter Teile zur Folge. Dadurch wird auch die Gefahr weiterer Brüche anderer Elemente bzw. weitere Brüche beider Schraubenfedern an anderen Stellen vermieden. Andererseits wird die Anzahl der benötigten Bauteile im Vergleich zu einer Federanordnung, wie sie aus dem Stand der Technik bekannt ist, insbesondere für große Federwege deutlich reduziert, da nicht mehr eine große Anzahl separater und einzeln zu fertigender und zu montierender Tellerfedern verwendet werden muss. Es müssen unabhängig von der benötigten Länge des jeweiligen Federelementes lediglich zwei Schraubenfedern ineinander geschraubt werden, wodurch das Herstellungsverfahren beschleunigt und gleichzeitig die Herstellungskosten gesenkt werden.

In einer bevorzugten Ausgestaltung des Gelenkes weist das Gelenk wenigstens zwei Federelemente auf, sodass das zweite Element in zwei einander gegenüberliegenden, also entgegen gesetzten, Richtungen gegen eine von wenigstens einen der wenigstens zwei Federelemente aufgebrachte Kraft schwenkbar ist. Dadurch kann beispielsweise im Falle eines Knöchelgelenkes, das durch das Gelenk gemäß diesem Ausführungsbeispiel der vorliegenden Erfindung gebildet, unterstützt oder nachgebildet werden soll, sowohl die Plantarflexion als auch die Dorsalflexion federbelastet werden. Vorzugsweise weisen die wenigstens zwei Federelemente jeweils wenigstens zwei Schraubenfedern auf, die jeweils aus einem Federband mit einer längeren Querschnittsseite hochkant zur Federachse gewickelt sind und derart ineinander geschraubt sind, dass die längeren Querschnittsseiten einen in unterschiedliche Richtungen von 90° abweichenden Winkel gegen die Federachse aufweisen und dass die Federbänder aneinander anliegen. Dadurch können die durch diese Art des Federelementes erreichten Vorteile doppelt verwendet werden. Natürlich können auch mehr als zwei Schraubenfedern verwendet werden, die in einander geschraubt werden. Dadurch wird die Federhärte bei nahezu gleichbleibendem Bauraum weiter erhöht.

Als Vorteilhaft hat sich herausgestellt, wenn die Federbänder zumindest teilweise aus einem Flachdraht oder einem Bandstahl bestehen. Das Federelement verfügt dann wenigstens teilweise, bevorzugt jedoch über seine gesamte Länge, über zwei gleiche ineinander geschraubte Schraubendruckfedern, von denen jede aus einem Bandstahl oder aus einem Flachdraht mit tellerfederähnlichem Querschnitt hochkant und schräg zur Mittelachse des Federelementes gewickelt ist. Der Querschnitt des verwendeten Bandstahles oder des verwendeten Flachdrahtes ist dabei in der verwendeten Schraubenfeder gegen die Längsachse der Schraubenfeder verkippt. Vorteilhafterweise werden die ineinander geschraubten Schraubenfedern so verwendet, dass diese Verkippung gegen die Längsachse der jeweiligen Schraubenfeder in unterschiedliche Richtungen vorliegt. Auf diese Weise werden tellerfederähnliche Anlageflächen der einzelnen Schraubenfedern aneinander erreicht. Die Schraubenfedern können auch aus Titan oder anderen Metallen oder Legierungen, insbesondere mit oder ohne Eisen, Kohlenstoff (Carbon) oder Kunststoff hergestellt und aus unterschiedlichen oder gleichen Materialien bestehen.

In einer bevorzugten Ausgestaltung befindet sich in wenigstens einem der Federelemente ein Pufferelement, das insbesondere aus einem Elastomer, besonders bevorzugt aus einem Polyurethan-Elastomer, wie es beispielsweise unter der Bezeichnung "Eladur" käuflich zu erwerben ist, besteht. Das Pufferelement, das vorteilhafterweise zylinderförmig ausgebildet ist, wird in der bevorzugten Ausgestaltung entlang der Längsachse des Federelementes in die beiden miteinander verschraubten Schraubenfedern eingeführt. Es dient folglich als weiteres Dämpfungs- und Federelement, als Anschlag und als Führungsdorn. Alternativ oder zusätzlich dazu kann ein derartiges Pufferelement auch in Form eines Hohlzylinders und in anderer geometrischer Ausgestaltung außen um das jeweilige Federelement herum angeordnet werden. So ist beispielsweise die Anordnung von zwei, drei, vier oder mehr zylinderförmigen Pufferelementen über den Umfang des Federelementes verteilt an dessen Außenseite denkbar. Die Positionierung des Pufferelementes im Innern des Federelementes weist jedoch den Vorteil auf, dass dadurch kein zusätzlicher Bauraum benötigt wird. Es ist auch möglich, das Federelement in das Material des Pufferelementes einzugießen.

Über die Elastizität oder Härte des Pufferelementes, die je nach verwendetem Material über einen bestimmten Bereich nahezu frei wählbar ist, lässt sich die Pufferwirkung und gegebenenfalls die Härte eines Anschlages einstellen.

Als vorteilhaft hat sich herausgestellt, wenn das Pufferelement kürzer ist als das Federelement, in oder an dem es angeordnet ist. Auf diese Weise kann es in besonders bevorzugter Ausgestaltung als zusätzlicher Puffer für einen Anschlag dienen, um hier ein hartes Anschlagen zusätzlich zu verhindern. Der Anschlag kann auch diese Weise gedämpft werden. Derartige Ausgestaltungen haben zudem den Vorteil, dass die tatsächliche und exakte Position des Anschlages und damit also der maximal mögliche Verschwenkwinkel des zweiten Elementes, das beispielsweise ein Fußteil sein kann, relativ zum ersten Element, das beispielsweise ein Unterschenkelanteil sein kann, individuell auf den jeweiligen Patienten in besonders einfacher Weise auch von einem Orthopädietechniker eingestellt werden kann. Er muss lediglich das Pufferelement entsprechend kürzen, um den Anschlag und damit den jeweils möglichen maximalen Verschwenkwinkel in eine oder beiden Richtungen individuell einstellen zu können. Damit ist es auch möglich, beispielsweise im Laufe einer Therapie diesen maximal möglichen Schwenkwinkel und damit die Position des Anschlages zu verändern, indem beispielsweise einfach neue Pufferelemente verwendet werden.

Selbstverständlich kann das Pufferelement auch länger als das Federelement ausgebildet sein und beispielsweise auf einer Seite des Federelementes über das Federelement hinausragen. Vorzugsweise ist an dieser Stelle in dem Gelenk eine Ausnehmung vorgesehen, in die der herausragende Anteil des Pufferelementes eingeführt ist. Für die Anschlagswirkung, die Änderung der Federkennlinie sowie die Änderung der Steifigkeit der Feder ist lediglich die wirksame Länge des Pufferelementes, also die Länge des Teils des Pufferelementes, das sich im Bereich des Federelementes befindet, entscheidend. Über das Pufferelement ist es möglich, eine progressive Federkennlinie zu unterstützen, indem das Pufferelement beispielsweise beim Kontakt mit einem Anschlag in die Zwischenräume der jeweiligen Federelemente eintritt und so die Kennlinie verändert.

Befindet sich ein Pufferelement im Inneren des Federelementes ist es von Vorteil, wenn der Außendurchmesser des Pufferelementes genauso groß oder nahezu genauso groß ist, wie der Innendurchmesser des Federelementes. Dadurch wird das Pufferelement bei starker Belastung, durch die es verkürzt wird, in die Lücken und Hohlräume zwischen den Federbändern der beiden ineinander geschraubten Schraubenfedern hineingedrückt. Dadurch wird einerseits die Anschlagswirkung verbessert und die Steifigkeit des Federelementes erhöht und andererseits die Haltbarkeit des Federelements und die Lebensdauer erhöht.

Vorzugsweise verfügt das Gelenk über wenigstens eine Spannvorrichtung, mit der wenigstens eines der Federelemente vorspannbar ist. Vorzugsweise verfügt das Gelenk über eine Spannvorrichtung für jedes der verwendeten Federelemente.

Als besonders Vorteilhaft hat sich herausgestellt, wenn der Grad der Vorspannung oder die Vorspannkraft einstellbar ausgebildet ist. Dies kann beispielsweise über Spannelemente geschehen, die das verwendete Federelement bereits in einer Nullstellung des jeweiligen Gelenkes mit einer gewissen Kraft vorspannen. Verfügt das Gelenk über wenigstens zwei derartige Federelemente, deren Vorspannung einstellbar ist, lässt sich auf diese Weise auch die jeweilige Nullstellung des Gelenkes, also die Position, die das zweite Element relativ zum ersten Element einnimmt, wenn keine zusätzlichen externen Kräfte auf das Gelenk wirken, einstellen. Auch dies ist für therapeutische und/oder Rehabilitationszwecke durchaus von Vorteil.

In einer bevorzugten Ausführungsform des Gelenkes sind die wenigstens zwei Federelemente verschieden ausgebildet. So können beispielsweise unterschiedliche Schraubenfedern für die jeweiligen Federelemente verwendet werden, wobei es sich jedoch als Vorteilhaft herausgestellt hat, wenn innerhalb eines Federelementes identische Schraubenfedern ineinander geschraubt werden. Durch die Verwendung unterschiedlicher Federelemente ist es beispielsweise möglich, die Verschwenkung des zweiten Elementes relativ zum ersten Element in einer ersten Richtung leichter auszugestalten als in einer der ersten Richtung entgegengesetzten zweiten Richtung. Es können auch Federelemente unterschiedlicher Länge verwendet werden, wodurch beispielsweise der Federweg, um den das jeweilige Federelement beispielsweise zusammengedrückt werden kann, eingestellt und bestimmt werden kann. Dadurch lässt sich ebenfalls der maximal mögliche Verschwenkwinkel in dieser Richtung einstellen.

Wie bereits dargelegt ist das Gelenk vorteilhafterweise ein Knöchelgelenk für eine Beinorthese oder eine Knöchelorthese.

In einer bevorzugten Ausgestaltung des Gelenkes ist das wenigstens eine Federelement von einem Dämpfungsmaterial, insbesondere einem Elastomer, umgeben. Vorteilhafterweise ist es von diesem umgossen. Dadurch wird verhindert, dass die Schraubenfedern des wenigstens einen Federelementes "auf Block gefahren" werden. Dies bedeutet, dass die einzelnen Windungen der Schraubenfedern vollflächig aneinander anliegen, sodass eine weitere Kompression des wenigstens einen Federelementes nicht mehr möglich ist. Dadurch wird die Lebensdauer des wenigstens einen Federelementes stark verringert und die Wahrscheinlichkeit eines mechanischen Versagens erhöht. Das Dämpfungsmaterial, das sich folglich auch zwischen den einzelnen Windungen der Schraubenfedern innerhalb des wenigstens einen Federelementes befindet, hat vorteilhafterweise gummielastische Eigenschaften und ist beispielsweise ein Elastomer. Dadurch wird innerhalb des wenigstens einen Federelementes ein gedämpfter Anschlag erreicht, der einerseits die vollständige Komprimierung des wenigstens einen Federelementes verhindert und gleichzeitig die Nachteile eines Festanschlages, wie er in vielen orthopädischen Gelenken zum Einsatz kommt, verhindert. Derartige feste Anschläge bedingen schlechten Trage- und Laufkomfort, was durch die gedämpfte Anschlagsausgestaltung durch das Dämpfungsmaterial verhindert wird. Das wenigstens eine Federelement kann dabei vorteilhafterweise vollständig von einem Kunststoff oder einem Polymer, beispielsweise einem Elastomer, umgossen sein. Die Shore-Härte kann dabei innerhalb des Dämpfungsmaterials gleichbleibend oder variierend ausgebildet sein.

Vorzugsweise befindet sich in dem wenigstens einen Federelement jedoch wenigstens ein Kanal, der nicht mit dem Dämpfungsmaterial gefüllt ist. Vorzugsweise befindet sich in diesem Kanal das bereits beschriebene Pufferelement. Durch diese einfache Ausgestaltung kann der gedämpfte Anschlag individuell eingestellt werden, da einerseits der Kunststoff, beispielsweise der Elastomer, der das Dämpfungsmaterial bildet und andererseits ein Kunststoff, insbesondere ein Elastomer, der das wenigstens eine Pufferelement bildet, frei und individuell gewählt werden kann. Es können unterschiedliche Shore-Härten oder andere Eigenschaften, beispielsweise Elastizitäten gewählt werden. Selbstverständlich ist es auch möglich, den Kanal im Inneren des Federelementes vorzusehen, in dem sich kein Dämpfungsmaterial befindet, ohne dass sich ein Pufferelement in diesem Kanal befindet.

Vorteilhafterweise verfügen das Dämpfungsmaterial und das Material des Pufferelementes über unterschiedliche Shore-Härten.

Mit Hilfe der beiliegenden Figuren wird nachfolgend ein Ausführungsbeispiel der vorliegenden Erfindung näher erläutert.

Es zeigt,
- Fig. 1: - ein Gelenk gemäß einem ersten Ausführungsbeispiel der vorliegenden Erfindung,
- Fig. 2: - das Gelenk aus Figur 1 in einer Explosionsdarstellung,
- Fig. 3: - ein Gelenk gemäß einem zweiten Ausführungsbeispiel der vorliegenden Erfindung,
- Fig. 4: - ein Gelenk gemäß dem Ausführungsbeispiel aus Figur 3 in einer Explosionsdarstellung,
- Fig. 5 - 7: - Schnittdarstellungen zweier Schraubenfedern in unterschiedlichen Stadien bei der Herstellung eines Federelementes,
- Fig. 8a - 8d: - Querschnitte durch unterschiedliche Federelemente.
- Fig. 9 - 12: - unterschiedlich ausgestaltete Federelemente in jeweils einer Schnittdarstellung (links), einer Ansicht senkrecht zur Federachse (rechts unten) und einer Ansicht entlang der Federachse (rechts oben).

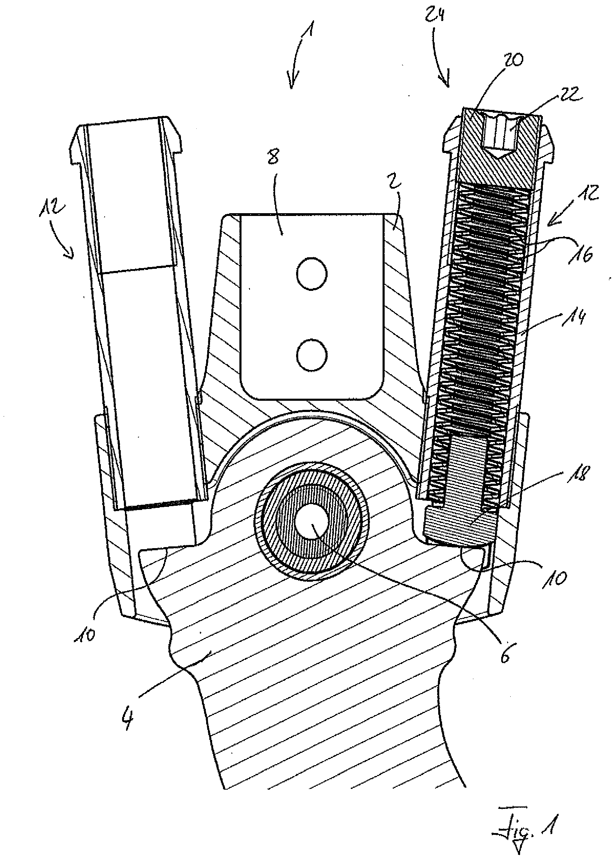

Figur 1 zeigt ein Gelenk 1 gemäß einem ersten Ausführungsbeispiel der vorliegenden Erfindung. Es verfügt über ein erstes Element 2 und ein zweites Element 4. Das zweite Element 4 ist um eine Schwenkachse 6 am ersten Element 2 schwenkbar gelagert. Das Gelenk 1 kann beispielsweise ein Knöchelgelenk sein. In diesem Fall bildet das zweite Element 4 einen Fußteil, während das erste Element 2 einen Unterschenkelanteil bildet. Man erkennt am ersten Element 2 eine Aufnahme 8, an der beispielsweise ein Schienenelement einer Orthese befestigt werden kann.

Das zweite Element 4 verfügt über zwei Anschlagselemente 10, die im gezeigten Ausführungsbeispiel als Schultern des zweiten Elementes 4 ausgebildet sind. Das Gelenk 1 verfügt über zwei Federelemente 12, von denen lediglich das rechte Federelement 12 dargestellt ist. Es befindet sich in einer Hülse 14, durch die es vor Verschmutzung geschützt und gleichzeitig geführt wird. Das Federelement 12 umfasst zwei Schraubenfedern 16, die ineinander geschraubt sind. Durch die Positionierung in der Hülse 14 und aufgrund der Eigenstabilität der Schraubenfedern 16 ist eine weitere Führung, beispielsweise durch einen Innendorn, nicht notwendig, kann jedoch in bestimmten Ausführungsformen von Vorteil sein.

Am in Figur 1 unteren Ende des Federelementes 12 befindet sich ein Gegenanschlagselement 18. Dieses liegt am Anschlagselement 10 des zweiten Elementes 4 an. Im oberen Bereich des Federelementes ist ein Schraubelement 20 vorhanden, das in ein Innengewinde der Hülse 14 eingeschraubt ist. Über eine in dem Schraubelement 20 vorhandene Vertiefung 22, in die ein Formschlusselement eingebracht werden kann, lässt sich das Schraubelement 20 relativ zur Hülse 14 drehen und somit weiter in die Hülse hinein oder aus der Hülse herausschrauben. Damit wird das Schraubelement 20 zusammen mit dem Innengewinde der Hülse 14 zu einer Spannvorrichtung 24. Wird das Schraubelement 20 weiter in die Hülse hineingeschraubt, werden die beiden Schraubenfedern 16 und damit das Federelement 12 zusammengedrückt. Dadurch wird die Vorspannung der Schraubenfedern 16 und des Federelementes 12 erhöht. Ein Verschwenken des zweiten Elementes 4 um die Schwenkachse 6 entgegen dem Uhrzeigersinn wird auf diese Weise erschwert.

Das in Figur 1 links dargestellte Federelement 12 ist vorteilhafterweise identisch ausgebildet, wobei es durchaus von Vorteil sein kann, andere Schraubenfedern 16 zu verwenden als dies im in Figur 1 rechts gezeigten Federelement 12 der Fall ist. Diese können sich in Material, Materialstärke, Windungsanzahl und/oder Länge unterscheiden.

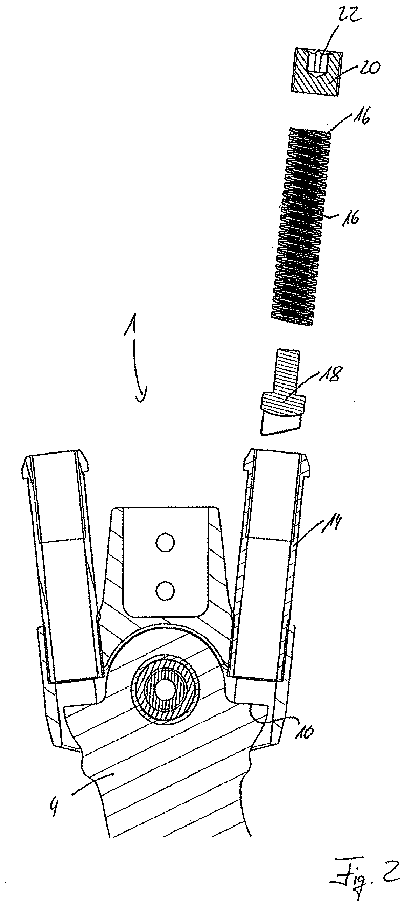

Figur 2 zeigt das Gelenk 1 in einer Explosionsdarstellung. Man erkennt die beiden Schraubenfedern 16, das Schraubelement 20 mit der Vertiefung 22 sowie die Hülse 14, in die diese Bauteile eingesetzt werden. Unterhalb der Schraubenfedern 16 ist das Gegenanschlagselement 18 dargestellt, das als Kontakt zum Anschlagselement 10 am zweiten Element 4 dient.

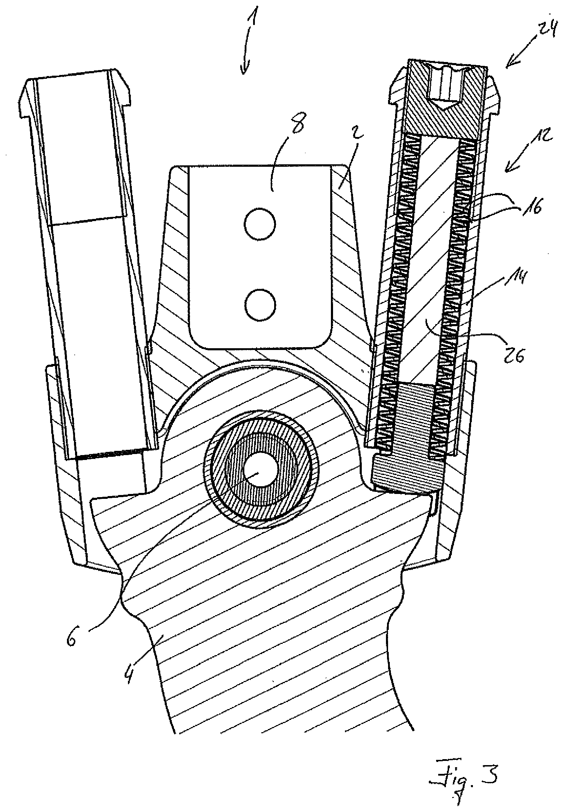

Figur 3 zeigt ein Gelenk 1 gemäß einem zweiten Ausführungsbeispiel der vorliegenden Erfindung in einer Darstellung gemäß Figur 1. Auch hier ist das erste Element 2 mit der Aufnahme 8 um die Schwenkachse 6 schwenkbar am zweiten Element 4 angeordnet. Das Federelement 12 mit den beiden Schraubenfedern 16 ist im Innern der Hülse 14 angeordnet und durch die Spannvorrichtung 24 vorspannbar. Anders als im in Figur 1 gezeigten Ausführungsbeispiel befindet sich im Innern des Federelementes nun ein Pufferelement 26, das als Führung, zusätzlicher Anschlag und zusätzliches Dämpfungselement wirkt. Die Länge des Pufferelementes 26 bestimmt, wann eine weitere Kompression des Federelementes 12 nicht mehr möglich und damit ein weiteres Verschwenken des zweiten Elementes 4 um die Schwenkachse 6 relativ zum ersten Element 2 ausgeschlossen ist. Auch hier lässt sich das zweite nicht dargestellte Federelement 12 im linken Teil der Figur 3 identisch oder verschieden zum Federelement 12 ausbilden. Insbesondere ist es möglich, lediglich in einem der beiden Federelemente 12 ein derartiges Pufferelement 26 vorzusehen.

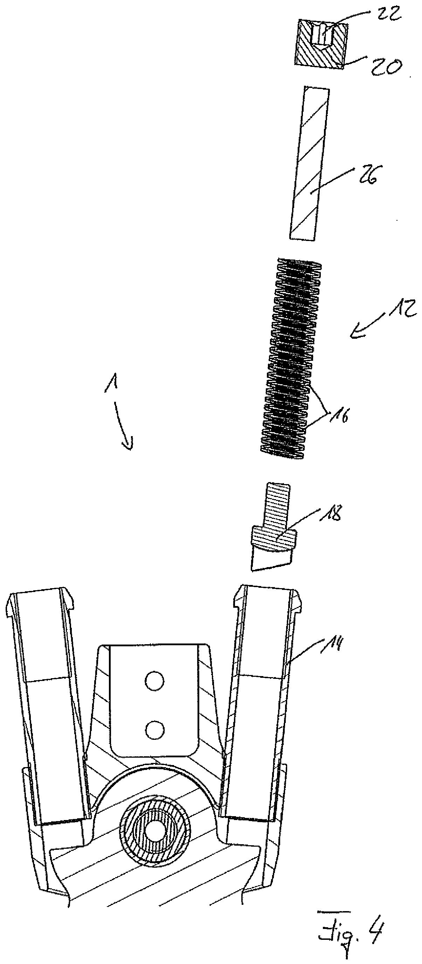

Figur 4 zeigt das Gelenk 1 aus Figur 3 in einer Explosionsdarstellung.

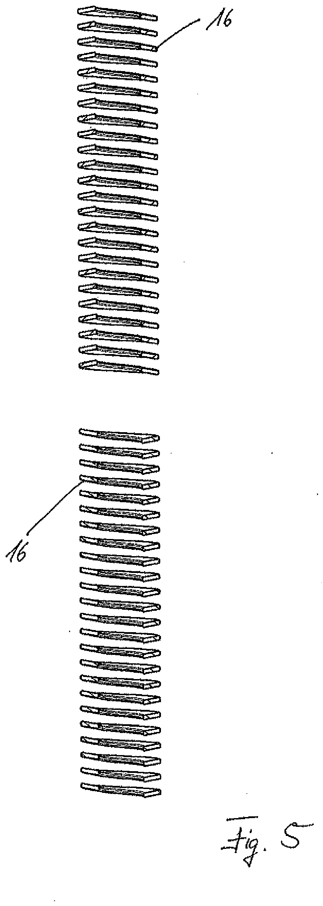

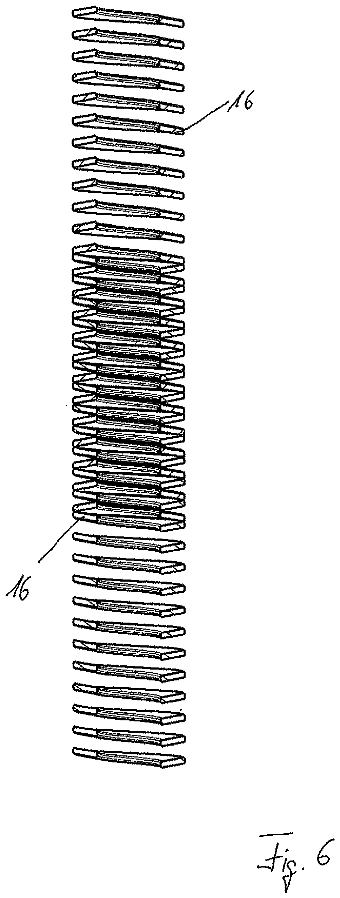

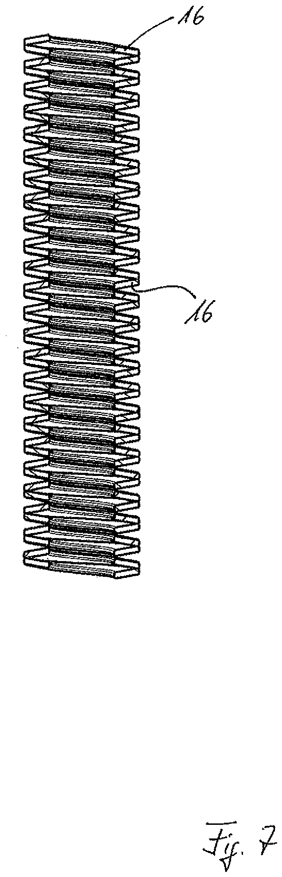

Figuren 5 bis 7 zeigen jeweils eine Schnittdarstellung zweier Schraubenfedern 16. Diese werden zum Herstellen eines Federelementes 12 in einander geschraubt. In Figur 5 sind die beiden Schraubenfedern 16 voneinander beabstandet dargestellt. Man erkennt, dass beide einen nahezu rechteckigen Querschnitt aufweisen und schräg zu einer Mittelachse, die in den Figuren 5 bis 7 von unten nach oben verläuft, gewickelt sind. Die langen Seiten des nahezu rechteckigen Querschnittes bilden folglich einen von 90° verschiedenen Winkel, wobei dieser Winkel für die beiden Schraubenfedern 16 in verschiedene Richtungen vom 90°-Winkel gegen die Mittelachse abweicht. Dadurch ergibt sich eine Positionierung der beiden Schraubenfedern 16 relativ zueinander, die der Anordnung bei separaten Tellerfedern ähnlich ist. Dies ist im mittleren Bereich der Figur 6 und in Figur 7 dargestellt, in denen die beiden Schraubenfedern 16 bereits in einander geschraubt sind.

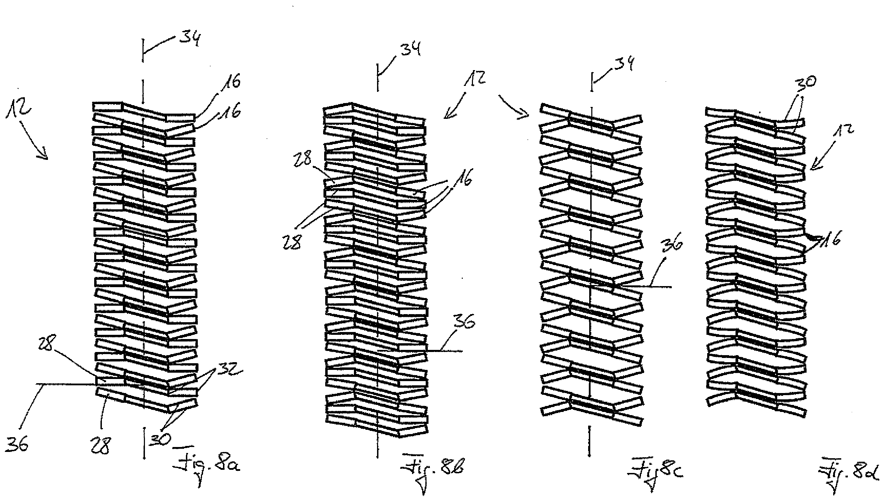

In den Figuren 8a bis 8d sind Querschnitte durch unterschiedliche Federelemente gezeigt.

Figur 8a zeigt ein Federelement 12, das aus zwei ineinander geschraubten Schraubenfedern 16 besteht. Jede dieser Schraubenfedern verfügt über einen Querschnitt 28, der rechteckig ausgebildet ist und somit zwei längere Querschnittsseiten 30 und zwei kürzere Querschnittsseiten 32 aufweist. In Figur 8a ist zudem eine Federachse 34 dargestellt. Auch eine Linie 36 ist zu erkennen, die zu der Federachse 34 im rechten Winkel verläuft.

Man erkennt, dass die eine der beiden Schraubenfedern 16 einen Querschnitt 28 aufweist, dessen längere Querschnittsseiten 30 exakt parallel zur Linie 36 verlaufen und damit einen Winkel von 90° zur Federachse 34 einschließt. Die längeren Querschnittsseiten 30 der zweiten Schraubenfeder 16 verlaufen hingegen in einem von 90° abweichenden Winkel zur Federachse 34. Die Querschnitte 28 der beiden Schraubenfedern 16 liegen abwechselnd radial innen und radial außen in einer linienförmigen Kontur aneinander an.

Figur 8b zeigt ein Federelement 12, das aus drei Schraubenfedern 16 hergestellt wurde. Auch hier sind die Querschnitte 28 rechteckig ausgebildet. Der Querschnitt 28 der mittleren Schraubenfeder 16 verfügt über längere Querschnittsseiten 30, die exakt senkrecht auf der Federachse 34 und damit parallel zur Linie 36 verlaufen. Die beiden anderen Schraubenfedern verfügen über Querschnitte 28, deren längere Querschnittsseiten 30 einen von 90° abweichenden Winkel mit der Federachse 34 einschließen. Dabei weichen die Winkel in unterschiedliche Richtungen von 90° ab. Daher bilden sich im Randbereich, also radial außen, Dreiergruppen von Querschnitten 28 der jeweiligen Schraubenfedern 16, die radial außen aneinander anliegen.

Figur 8c zeigt ein Federelement, das aus zwei Schraubenfedern 16 hergestellt wurde, die jeweils über Querschnitte 28 verfügen, die rechteckig ausgebildet sind. Die längeren Querschnittsseiten 30 verlaufen nicht parallel zur Linie 36 und bilden daher keinen rechten Winkel zur Federachse 34. Die Abweichungen von diesen 90° Winkel sind jedoch für unterschiedliche Schraubenfedern 16 unterschiedlich. Gleiches gilt für die in Figur 8d gezeigte Anordnung aus zwei Schraubenfedern 16, mit dem Unterschied, dass die längeren Querschnittsseiten 30 der beiden verwendeten Schraubenfedern nicht als gerade Linien, sondern gebogen oder gewölbt ausgebildet sind.

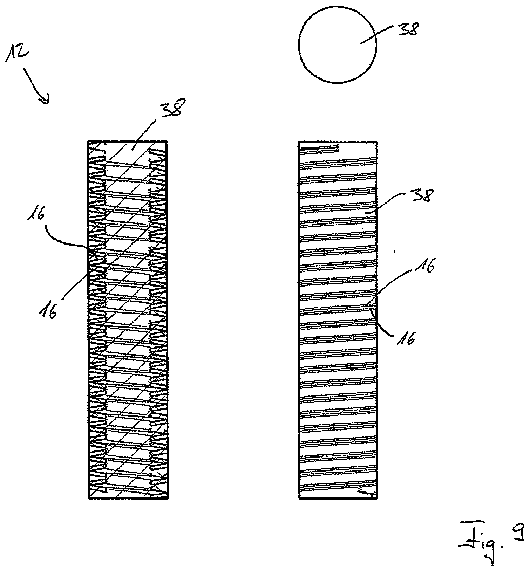

Figur 9 zeigt eine schematische Darstellung eines Federelementes 12 in unterschiedlichen Ansichten. Die links dargestellte Schnittdarstellung zeigt die beiden Schraubenfedern 16, die vollständig von einem Dämpfungsmaterial 38 umgeben sind. In der Seitenansicht senkrecht zur Federachse sind ebenfalls die Schraubenfedern 16 und das dazwischen liegende Dämpfungsmaterial 38 zu erkennen. Rechts oben ist eine schematische Ansicht entlang der Federachse dargestellt, an der lediglich das Dämpfungsmaterial 38 zu erkennen ist.

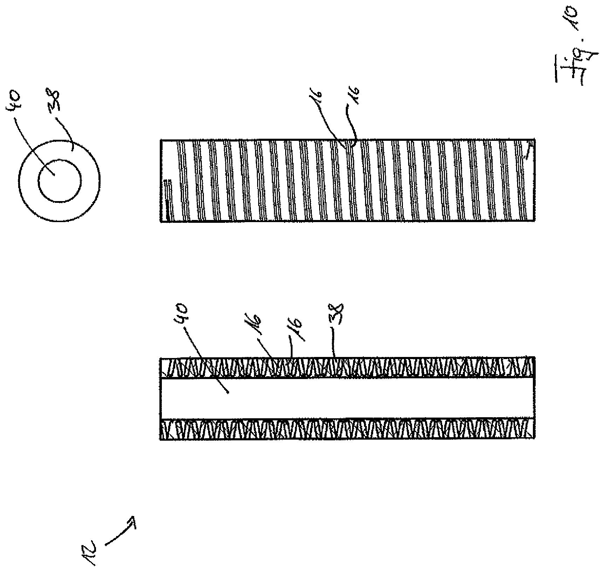

Figur 10 zeigt die gleichen Darstellungen wie Figur 9 mit einem geänderten Federelement 12. Auch hier sind die beiden Schraubenfedern 16 vom Dämpfungsmaterial 38 umgeben. Allerdings befindet sich im Inneren der Schraubenfedern 16 ein Kanal 40, der nicht mit Dämpfungsmaterial 38 gefüllt ist. Die Ansicht senkrecht zur Federachse rechts unten in Figur 10 entspricht der in Figur 9 gezeigten Ansicht, da die in Figur 9 und in Figur 10 gezeigte Ausführungsform des jeweiligen Federelementes 12 in dieser Ansicht identisch aussieht. Rechts oben in der Darstellung parallel zur Federachse ist jedoch das Dämpfungsmaterial 38 und der sich zentral darin befindende Kanal 40 zu erkennen. Unabhängig von der Ausgestaltung ist es nicht notwendig, dass der Kanal 40 sich durch das gesamte Federelement 12 erstreckt. Es ist auch möglich, dass der Kanal 40 beispielsweise nur auf einer Seite eine Öffnung aufweist.

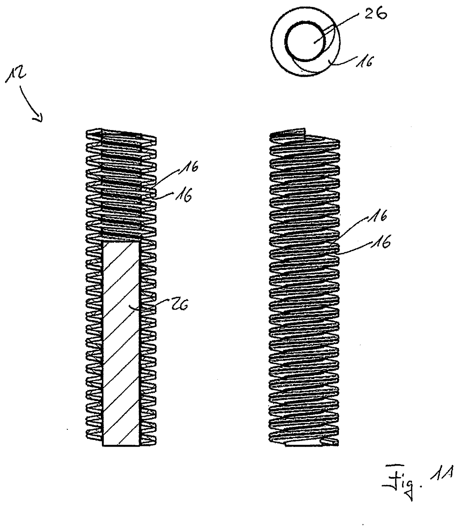

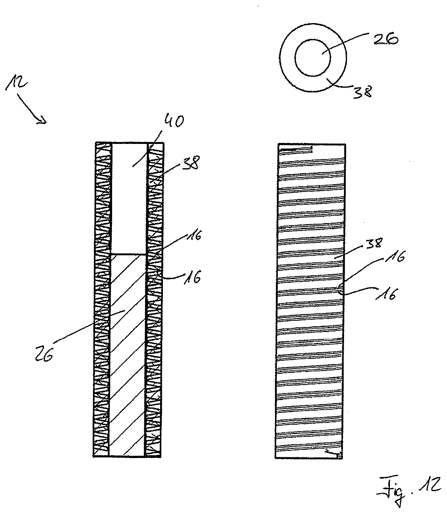

Figur 11 zeigt die gleiche Darstellung wie in den Figuren 9 und 10 mit einem weiteren Federelement 12. Die beiden Schraubenfedern 16 sind zu erkennen und nicht von einem Dämpfungsmaterial 38 umgeben. Im Inneren der Schraubenfedern 16 befindet sich das Pufferelement 26, das insbesondere auch in der Darstellung parallel zur Federachse zu erkennen ist. In Figur 12 ist die Darstellung des in Figur 10 gezeigten Federelementes 12 dargestellt, wobei sich nun im Inneren des Kanals 40 das Pufferelement 26 befindet.

### Bezugszeichenliste

- 1: Gelenk
- 2: erstes Element
- 4: zweites Element
- 6: Schwenkachse
- 8: Aufnahme
- 10: Anschlagselement
- 12: Federelement
- 14: Hülse
- 16: Schraubenfeder
- 18: Gegenanschlagselement
- 20: Schraubelement
- 22: Vertiefung
- 24: Spannvorrichtung
- 26: Pufferelement
- 28: Querschnitt
- 30: längere Querschnittsseite
- 32: kürzere Querschnittsseite
- 34: Federachse
- 36: Linie
- 38: Dämpfungsmaterial
- 40: Kanal

## Patentansprüche

1. Gelenk (1) für eine orthopädietechnische Einrichtung, insbesondere eine Orthese oder Prothese, wobei das Gelenk (1)
- ein erstes Element (2),
- wenigstens ein Federelement (12) und
- ein zweites Element (4) aufweist, das an dem ersten Element (2) entgegen einer von dem wenigstens einen Federelement (12) in wenigstens einer Richtung aufgebrachten Kraft schwenkbar gelagert ist,
**dadurch gekennzeichnet, dass** das wenigstens eine Federelement (12) wenigstens zwei Schraubenfedern (16) aufweist,
- die jeweils aus einem Federband mit einer längeren Querschnittsseite hochkant zur Federachse gewickelt sind und
- derart ineinander geschraubt sind,
- dass die längere Querschnittsseite wenigstens einer der Schraubenfedern (16) einen von 90° abweichenden Winkel gegen die Federachse aufweist und
- dass die Federbänder aneinander anliegen.

2. Gelenk (1) nach Anspruch 1, **dadurch gekennzeichnet, dass** das Gelenk (1) wenigstens zwei Federelemente (12) aufweist, sodass das zweite Element (4) in zwei entgegengesetzten Richtungen gegen eine von wenigstens einem der wenigstens zwei Federelemente (12) aufgebrachte Kraft schwenkbar ist.

3. Gelenk (1) nach Anspruch 2, **dadurch gekennzeichnet, dass** die wenigstens zwei Federelemente (12) jeweils wenigstens zwei Schraubenfedern (16) aufweisen, die jeweils aus einem Federband mit einer längeren Querschnittsseite hochkant zur Federachse gewickelt sind und derart ineinander geschraubt sind, dass die längeren Querschnittsseiten einen in unterschiedliche Richtungen von 90° abweichenden Winkel gegen die Federachse aufweisen und dass die Federbänder aneinander anliegen.

4. Gelenk (1) nach einem der vorstehenden Ansprüche, **dadurch gekennzeichnet, dass** die Federbänder zumindest teilweise aus einem Flachdraht oder einem Bandstahl bestehen.

5. Gelenk (1) nach einem der vorstehenden Ansprüche, **dadurch gekennzeichnet, dass** sich in wenigstens einem Federelement (12) ein Pufferelement (26), insbesondere aus einem Elastomer, besonders bevorzugt aus einem Polyurethan-Elastomer, befindet.

6. Gelenk (1) nach Anspruch (5), **dadurch gekennzeichnet, dass** das Pufferelement (26) kürzer ist als das Federelement (12), in dem es angeordnet ist.

7. Gelenk (1) nach einem der vorstehenden Ansprüche, **dadurch gekennzeichnet, dass** das Gelenk (1) über wenigstens eine Spannvorrichtung (24) verfügt, mit der wenigstens ein Federelement (12) vorspannbar ist.

8. Gelenk (1) nach Anspruch 7, **dadurch gekennzeichnet, dass** die Vorspannung einstellbar ist.

9. Gelenk (1) nach einem der Ansprüche 2 bis 8, **dadurch gekennzeichnet, dass** die wenigstens zwei Federelemente (12) verschieden ausgebildet sind.

10. Gelenk (1) nach einem der vorstehenden Ansprüche, **dadurch gekennzeichnet, dass** das Gelenk (1) ein Knöchelgelenk für eine Beinorthese oder eine Knöchelorthese ist.

11. Gelenk (1) nach einem der vorstehenden Ansprüche, **dadurch gekennzeichnet, dass** das wenigstens eine Federelement (12) von einem Dämpfungsmaterial (38), insbesondere einem Elastomer, umgeben, insbesondere von diesem umgossen ist.

12. Gelenk (1) nach Anspruch 11, **dadurch gekennzeichnet, dass** sich in dem wenigstens einen Federelement (12) wenigstens ein Kanal (40) befindet, der nicht mit Dämpfungsmaterial (38) gefüllt ist, wobei sich in dem wenigstens einen Kanal (40) bevorzugt das Pufferelement (26) befindet.

13. Gelenk (1) nach Anspruch 12, **dadurch gekennzeichnet, dass** das Dämpfungsmaterial (38) und das Material des Pufferelementes (26) unterschiedliche Shore-Härten aufweisen.

## Claims

1. Joint (1) for an orthopedic device, in particular an orthosis or prosthesis, wherein the joint (1) has
- a first element (2),
- at least one spring element (12), and
- a second element (4) which is mounted pivotably on the first element (2) counter to a force applied by the at least one spring element (12) in at least one direction,
**characterized in that** the at least one spring element (12) has at least two helical springs (16)
- which are each wound from a spring strip having a longer cross-sectional side edgeways with respect to the spring axis and
- which are screwed into each other in such a way that
- the longer cross-sectional side of at least one of the helical springs (16) has an angle to the spring axis deviating from 90°, and
- the spring strips bear on each other.

2. Joint (1) according to Claim 1, **characterized in that** the joint (1) has at least two spring elements (12), such that the second element (4) is pivotable in two opposite directions counter to a force applied by at least one of the at least two spring elements (12).

3. Joint (1) according to Claim 2, **characterized in that** the at least two spring elements (12) each have at least two helical springs (16) which are each wound from a spring strip having a longer cross-sectional side edgeways with respect to the spring axis and which are screwed into each other in such a way that the longer cross-sectional sides have an angle deviating from 90° relative to the spring axis in different directions, and such that the spring strips bear on each other.

4. Joint (1) according to one of the preceding claims, **characterized in that** the spring strips are made at least partially from a flat wire or a steel strip.

5. Joint (1) according to one of the preceding claims, **characterized in that** a buffer element (26), in particular made of an elastomer, particularly preferably made of a polyurethane elastomer, is located in at least one spring element (12).

6. Joint (1) according to Claim 5, **characterized in that** the buffer element (26) is shorter than the spring element (12) in which it is arranged.

7. Joint (1) according to one of the preceding claims, **characterized in that** the joint (1) has at least one tensioning device (24), with which at least one spring element (12) can be pretensioned.

8. Joint (1) according to Claim 7, **characterized in that** the pretensioning is adjustable.

9. Joint (1) according to one of Claims 2 to 8, **characterized in that** the at least two spring elements (12) are configured differently.

10. Joint (1) according to one of the preceding claims, **characterized in that** the joint (1) is an ankle joint for a leg orthosis or an ankle orthosis.

11. Joint (1) according to one of the preceding claims, **characterized in that** the at least one spring element (12) is surrounded by a damping material (38), in particular an elastomer, in particular encapsulated by the latter.

12. Joint (1) according to Claim 11, **characterized in that** at least one channel (40), which is not filled with damping material (38), is located in the at least one spring element (12), wherein the buffer element (26) is preferably located in the at least one channel (40).

13. Joint (1) according to Claim 12, **characterized in that** the damping material (38) and the material of the buffer element (26) have different Shore hardnesses.

## Revendications

1. Articulation (1) pour un dispositif orthopédique, en particulier pour une orthèse ou une prothèse, l'articulation (1) comportant
- un premier élément (2),
- au moins un élément ressort (12), et
- un second élément (4) monté sur le premier élément (2) de façon mobile en pivotement à l'encontre d'une force exercée par ledit au moins un élément ressort (12) dans au moins une direction,
**caractérisée en ce que**
ledit au moins un élément ressort (12) comprend au moins deux ressorts hélicoïdaux (16) qui
- sont enroulés chacun à partir d'une bande élastique ayant une face de section transversale plus longue de chant par rapport à l'axe de ressort et qui
- sont vissés l'un dans l'autre de telle sorte que
- la face de section transversale plus longue de l'un au moins des ressorts hélicoïdaux (16) présente un angle différent de 90° par rapport à l'axe de ressort, et que
- les bandes élastiques s'appuient l'une contre l'autre.

2. Articulation (1) selon la revendication 1,
**caractérisée en ce que**
l'articulation (1) comprend au moins deux éléments ressorts (12), de sorte que le second élément (4) est mobile en pivotement à l'encontre d'une force exercée par au moins un desdits au moins deux éléments ressorts (12) dans deux directions opposées.

3. Articulation (1) selon la revendication 2,
**caractérisée en ce que**
lesdits au moins deux éléments ressorts (12) présentent chacun au moins deux ressorts hélicoïdaux (16) qui sont enroulés chacun à partir d'une bande élastique ayant une face de section transversale plus longue de chant par rapport à l'axe de ressort et qui sont vissés l'un dans l'autre de telle sorte que les faces de section transversale plus longues présentent un angle différent de 90° par rapport à l'axe de ressort dans différentes directions, et que les bandes élastiques s'appuient l'une contre l'autre.

4. Articulation (1) selon l'une des revendications précédentes,
**caractérisée en ce que**
les bandes élastiques sont constituées au moins partiellement d'un fil plat ou d'un acier en bande.

5. Articulation (1) selon l'une des revendications précédentes,
**caractérisée en ce que**
un élément tampon (26), en particulier en un élastomère, de préférence en un élastomère de polyuréthane, se trouve dans au moins un élément ressort (12).

6. Articulation (1) selon la revendication 5,
**caractérisée en ce que**
l'élément tampon (26) est plus court que l'élément ressort (12) dans lequel il est agencé.

7. Articulation (1) selon l'une des revendications précédentes,
**caractérisée en ce que**
l'articulation dispose d'au moins un dispositif de serrage (24) permettant de mettre sous précontrainte au moins un élément ressort (12).

8. Articulation (1) selon la revendication 7,
**caractérisée en ce que**
la précontrainte est réglable.

9. Articulation (1) selon l'une des revendications 2 à 8,
**caractérisée en ce que**
lesdits au moins deux éléments ressorts (12) sont réalisés différemment.

10. Articulation (1) selon l'une des revendications précédentes,
**caractérisée en ce que**
l'articulation (1) est une articulation de cheville pour une orthèse de jambe ou une orthèse de cheville.

11. Articulation (1) selon l'une des revendications précédentes,
**caractérisée en ce que**
ledit au moins un élément ressort (12) est entouré, en particulier surmoulé, d'un matériau d'amortissement (38), en particulier d'un élastomère.

12. Articulation (1) selon la revendication 11,
**caractérisée en ce que**
au moins un canal (40) se trouve dans ledit au moins un élément ressort (12), qui n'est pas rempli du matériau d'amortissement (38), l'élément tampon (26) se trouvant de préférence dans ledit au moins un canal (40).

13. Articulation (1) selon la revendication 12,
**caractérisée en ce que**
le matériau d'amortissement (38) et le matériau de l'élément tampon (26) présentent différentes duretés Shore.
